# EUROPEAN PATENT APPLICATION

(11) **EP 3 901 276 A1**
(43) Date of publication of application: **27.10.2021**
(21) Application number: 20171338.5
(22) Date of filing: 24.04.2020
(51) Int. Cl.: C12Q 1/54, G01N 33/569, G01N 33/66, G01N 33/68

(54) **METHOD AND DEVICE FOR ASSESSING A STATUS OF A WOUND OF A PATIENT**

(71) Applicant: Paul Hartmann AG, 89522 Heidenheim (DE)
(72) Inventor: SMOLA, Hans, 66121 Saarbrücken (DE); JORGE, Ana, 71640 Ludwigsburg (DE)

(57) **Abstract**

The present invention relates to a method for assessing a status of a wound of a patient, comprising the steps of
i. determining a concentration of one or more inflammatory markers selected from glucose and eotaxin-1 in a sample from the wound, wherein the sample comprises or consists of wound fluid and the one or more inflammatory markers may indicate an inflammation within the wound,
ii. determining a concentration of one or more bacterial markers in the sample, wherein the one or more bacterial markers may indicate a colonization of the wound with metabolically active bacteria,
iii. assessing the status of the wound based on the concentrations of the one or more inflammatory markers and the one or more bacterial markers, wherein the status of the wound is assessed as being inflamed or not inflamed and as being colonized with metabolically active bacteria or not colonized with metabolically active bacteria.
This diagnostic method allows a reliable, specific and detailed assessment of the status of the wound.

## Description

The present invention relates to a method for assessing the status of a wound of a patient. Moreover, the present invention relates to a corresponding device for assessing the status of a wound of a patient.

In acute and chronic wounds, prolonged and overshooting inflammation interferes with normal wound healing processes. This uncontrolled inflammation might be the result of several underlying diseases treated in different ways. The most common is bacterial colonization, which can progress to local and, in severe cases, to systemic infection. Here, antibiotic treatment is recommended. Uncontrolled, overshooting host immune responses may also trigger inflammation. In this case, immunosuppressive therapies are advised to support healing. The identification of an infected wound is not easy to the naked eye, leading to inappropriate treatments with consequences for healing. Current methods to ascertain infection include interpretation of clinical wound symptoms, swabs and culture of the bacteria, which delays the initiation of the appropriate treatment. In many clinical situations health care professionals are often confronted with wound conditions, where the diagnosis of whether a wound is infected or not is difficult to make. The consequence is often overuse of systemic antibiotics, which can lead to emergence and spread of antibiotic resistance in bacteria (Lipsky et al. Antimicrobial stewardship in wound care: a Position Paper from the British Society for Antimicrobial Chemotherapy and European Wound Management Association. J Antimicrob Chemother. 2016, 71:3026-3035).

Improved diagnostic methods to identify an infected wound are also described in the patent literature. For example, WO 2003/040406 A2 discloses a method of diagnosing clinical infection of a wound at an early inflammatory stage, wherein the concentration of a marker associated with an inflammatory response is measured in wound fluid. The marker is a fibronectin fragment, a neutrophil protease or a macrophage protease. In particular, WO'406 proposes to use neutrophil elastase for diagnosing a wound infected with bacteria. Similarly, WO 2004/086043 A1 suggests to measure a proinflammatory cytokine, preferably TNF-α, in wound fluid to identify an infected wound. If such a marker is measured in the wound fluid in elevated concentration, an antimicrobial treatment of the wound is indicated according to the disclosure of the aforementioned international patent applications. However, there might be circumstances in which wound fluid contains an inflammatory marker at an elevated level, even though the wound is not infected and consequently does not require antimicrobial treatment.

The present invention aims to provide a novel diagnostic method and diagnostic device, which allows assessment of the wound status in a reliable, specific, and detailed manner. This is achieved by a method according to claim 1 and a device according to claim 14.

As already mentioned, the present invention deals with a method for assessing a status of a wound of a patient. The patient is an animal or in particular a human being. The method may be used for assessing different wound types. For example, the wound may be an acute wound such as an acute traumatic wound or a chronic wound such as a diabetic ulcer, decubitus ulcer or leg ulcer. The method according to the present invention comprises three steps, which are described in the following.

In the first step (step i.) of the method, a concentration of one or more inflammatory markers selected from glucose and eotaxin-1 in a sample from the wound is determined. If more than one inflammatory marker is determined in the sample (that is glucose and eotaxin-1), the concentration of each single inflammatory marker is normally determined instead of just determining the total concentration of the inflammatory markers in the sample. The sample comprises or preferably consists of wound fluid. Importantly, the one or more inflammatory markers may indicate an inflammation within the wound and originate from the patient.

In the second step (step ii.) of the method, a concentration of one or more bacterial markers in the sample from the wound is determined. Again, if more than one bacterial marker is determined in the sample, the concentration of each single bacterial marker is normally determined instead of just determining the total concentration of the bacterial markers in the sample. Importantly, the one or more bacterial markers may indicate a colonization of the wound with metabolically active bacteria. As the designation "bacterial markers" implies these markers originate from bacteria and not from the patient. Metabolically active bacteria are bacteria which are alive and somehow metabolically active. They are distinguished from dead bacteria and bacteria which are alive, but metabolically inactive. Typically, markers which may stem from inactive or in particular dead bacteria are no bacterial markers in the sense of the present invention.

The sequence of the first and the second step is not essential and may also be changed. Therefore, instead of at first determining the inflammatory markers and subsequently determining the bacterial markers, it may likewise be possible to at first determine the bacterial markers and afterwards determine the inflammatory markers. Depending on the technical implementation of the present diagnostic method, the first and the second step may even take place at the same time.

In the third step (step iii.) of the method, the status of the wound based on the determined concentrations of the one or more inflammatory markers and the one or more bacterial markers is assessed. The status of the wound is assessed as being inflamed or not inflamed and as being colonized with metabolically active bacteria or not colonized with metabolically active bacteria. Therefore, the assessment of the wound based on the determined concentrations of the markers leads to the conclusion if the wound is inflamed or not and furthermore if the wound is colonized with metabolically active bacteria or not.

Wound fluid - often also designated as exudate or wound exudate - is typically a fluid (liquid) that is secreted by the wound and which composition generally reflect the inflammation and colonization status of the wound. Wound fluid may amongst others also contain cells of the patient and bacteria. Preferably, wound fluid does substantially not contain blood.

In particular, the sample with the wound fluid may be removed from the wound and the body of the patient before the method of the present invention is performed. In this case the presence of the patient is not required to perform the method of the present invention. For instance, the sample may be removed by aspiration (for example with a pipette or a negative pressure wound therapy system) or absorption (for example with a swab or test strip). Alternatively, the method of the present invention or at least step i. and ii. of the method of the present invention may be performed on the body of the patient (in situ). The wound dressing mentioned below may be an example where the method according to the present invention may be performed in situ.

In the first and the second step usually the free concentration of the markers in the sample is determined. Thus, substances within intact cells and intact bacteria possibly contained in the sample are usually not determined in step i. and ii. of the present method. Besides, the term "determining a concentration" may also include the sole determination and/or indication whether a marker is present or absent in the sample.

The method according to claim 1 has several advantages. As pointed out before, the method determines inflammatory markers of the patient and in addition bacterial markers of metabolically active bacteria in the wound fluid. This allows a reliable, specific and detailed assessment of the status of the wound, namely whether the wound is inflamed or not as well as whether the wound is colonized with metabolically active bacteria or not. In this way the diagnostic method of the present invention may eventually improve the wound treatment and the wound healing, since the type of wound treatment is chosen based on the assessed or assumed wound status. The suggested diagnostic method is thus a useful tool to identify the correct wound treatment and to exclude unnecessary or even detrimental wound treatments. This will be explained in the following in more detail. Furthermore, the diagnostic method and device of the present invention allows an easy and quick wound status assessment.

According to the invention, the inflammatory markers are selected from glucose and eotaxin-1. Therefore, as inflammatory marker(s) either glucose, eotaxin-1 or glucose and eotaxin-1 may be used. Glucose as well as eotaxin-1 may indicate an inflammation within the wound if they are present in the wound fluid in decreased amounts. Eotaxin-1 is also called CC-chemokine ligand 11 (CCL11). In a preferred embodiment of the present invention the inflammatory marker is glucose.

In another preferred embodiment of the present invention, the one or more bacterial markers are selected from a coagulase, an enzyme, in particular an active enzyme, a metabolite, a siderophore, a signaling molecule and a virulence factor.
The coagulase may be staphylocoagulase, von Willebrand factor binding protein (vWbp), clumping factor A (ClfA) or clumping factor B (ClfB). In particular, the coagulase may be staphylocoagulase.
The enzyme may be alkaline phosphatase or nitrate reductase.
The metabolite may be acetoin, acetone, ammonia, nitrate or nitrite. In particular, the metabolite may be acetoin, acetone, ammonia or nitrite.
The siderophore may be acinetobactin, aerobactin, baumannoferrin, enterobactin, fimsbactin, staphyloferrin A, staphyloferrin B, pyochelin, pyoverdine, salmochelin or yersiniabactin. In particular, the siderophore may be pyoverdine.
The signaling molecule may be a quorum sensing molecule. In particular, the signaling molecule may be indole or autoinducer-2 (AI-2). Indole as well as autoinducer-2 both belong to the class of quorum sensing molecules.
The virulence factor may be a quorum sensing regulated molecule. In particular, the virulence factor may be alkaline protease, alpha-toxin, γ-hemolysin, LasA protease, LasB elastase, LecA lectin, LukDE, LukGH (LukAB), panton-valentine-leukocidin (PVL), a phenol soluble modulin (PSM), pyocyanin or a rhamnolipid. These examples for the virulence factor are all quorum sensing regulated molecules, that is they are regulated by quorum sensing. A particular preferred virulence factor is pyocyanin.
All bacterial markers mentioned above may indicate a colonization of the wound with metabolically active bacteria if they are present in the wound fluid. Nevertheless, the metabolites, for example acetoin, acetone, ammonia or nitrite, are particularly preferred bacterial markers to detect metabolically active bacteria in the wound.

The following **table 1** lists possible groups from which the marker(s) may be selected. For example, according to the embodiment mentioned in row 5 of table 1 the one or more inflammatory markers are selected from glucose and eotaxin-1, or the inflammatory marker is preferably only glucose. In addition, in the embodiment of row 5 the one or more bacterial markers are selected from acetoin, acetone, ammonia, nitrate and nitrite, or preferably only from acetoin, acetone, ammonia and nitrite.

**Table 1: possible groups from which the marker(s) may be selected**

| | **inflammatory marker(s) selected from** | **bacterial marker(s) selected from** |
|---|---|---|
| 1 | glucose and eotaxin-1, in particular glucose | a coagulase, preferably staphylocoagulase, von Willebrand factor binding protein (vWbp), clumping factor A (ClfA) or clumping factor B (ClfB), an enzyme, preferably alkaline phosphatase or nitrate reductase, a metabolite, preferably acetoin, acetone, ammonia, nitrate or nitrite, a siderophore, preferably acinetobactin, aerobactin, baumannoferrin, enterobactin, fimsbactin, staphyloferrin A, staphyloferrin B, pyochelin, pyoverdine, salmochelin or yersiniabactin, a signaling molecule, preferably a quorum sensing molecule (in particular indole or autoinducer-2), and a virulence factor, preferably a quorum |
| | | sensing regulated molecule (in particular alkaline protease, alpha-toxin, γ-hemolysin, LasA protease, LasB elastase, LecA lectin, LukDE, LukGH (LukAB), panton-valentine-leukocidin (PVL), a phenol soluble modulin (PSM), pyocyanin or a rhamnolipid) |
| 2 | glucose and eotaxin-1, in particular glucose | staphylocoagulase, von Willebrand factor binding protein (vWbp), clumping factor A (ClfA), clumping factor B (ClfB), alkaline phosphatase, nitrate reductase, acetoin, acetone, ammonia, nitrate, nitrite, acinetobactin, aerobactin, baumannoferrin, enterobactin, fimsbactin, staphyloferrin A, staphyloferrin B, pyochelin, pyoverdine, salmochelin, yersiniabactin, a quorum sensing molecule (in particular indole or autoinducer-2), and a quorum sensing regulated molecule (in particular alkaline protease, alpha-toxin, γ-hemolysin, LasA protease, LasB elastase, LecA lectin, LukDE, LukGH (LukAB), panton-valentine-leukocidin (PVL), a phenol soluble modulin (PSM), pyocyanin or a rhamnolipid) |
| 3 | glucose and eotaxin-1, in particular glucose | staphylocoagulase, von Willebrand factor binding protein (vWbp), clumping factor A (ClfA), clumping factor B (ClfB), alkaline phosphatase, nitrate reductase, acetoin, acetone, ammonia, nitrate, nitrite, acinetobactin, aerobactin, baumannoferrin, enterobactin, fimsbactin, staphyloferrin A, staphyloferrin B, pyochelin, pyoverdine, salmochelin, |
| | | yersiniabactin, indole, autoinducer-2, alkaline protease, alpha-toxin, γ-hemolysin, LasA protease, LasB elastase, LecA lectin, LukDE, LukGH (LukAB), panton-valentine-leukocidin (PVL), a phenol soluble modulin (PSM), pyocyanin, and a rhamnolipid |
| 4 | glucose and eotaxin-1, in particular glucose | staphylocoagulase, alkaline phosphatase, nitrate reductase, acetoin, acetone, ammonia, nitrite, pyoverdine and pyocyanin |
| 5 | glucose and eotaxin-1, in particular glucose | acetoin, acetone, ammonia, nitrate and nitrite, in particular acetoin, acetone, ammonia and nitrite |

The following **table 2** lists preferred embodiments of specific marker combinations. For example, according to the embodiment mentioned in row 2 of table 2 the inflammatory marker is glucose and the bacterial marker is alkaline phosphatase.

**Table 2: specific marker combinations**

| | **inflammatory marker** | **bacterial marker** |
|---|---|---|
| 1 | glucose | staphylocoagulase |
| 2 | glucose | alkaline phosphatase |
| 3 | glucose | nitrate reductase |
| 4 | glucose | acetoin |
| 5 | glucose | acetone |
| 6 | glucose | ammonia |
| 7 | glucose | nitrite |
| 8 | glucose | pyoverdine |
| 9 | glucose | pyocyanin |
| 10 | eotaxin-1 | staphylocoagulase |
| 11 | eotaxin-1 | alkaline phosphatase |
| 12 | eotaxin-1 | nitrate reductase |
| 13 | eotaxin-1 | acetoin |
| 14 | eotaxin-1 | acetone |
| 15 | eotaxin-1 | ammonia |
| 16 | eotaxin-1 | nitrite |
| 17 | eotaxin-1 | pyoverdine |
| 18 | eotaxin-1 | pyocyanin |

According to the invention the status of the wound is assessed based on the determined concentrations of the one or more inflammatory markers and the one or more bacterial markers to conclude whether the wound is inflamed or not and furthermore whether the wound is colonized with metabolically active bacteria or not. Typically, for this assessment the determined concentrations of the one or more inflammatory markers and the one or more bacterial markers are compared with predetermined concentrations for the one or more inflammatory markers and the one or more bacterial markers. That is, every concentration which has been determined for a particular inflammatory or bacterial marker is typically compared with a predetermined concentration for this particular inflammatory or bacterial marker. This, however, does not exclude the possibility that the predetermined concentration for one particular marker also applies for another marker. The predetermined concentrations for the inflammatory markers may be understood as limits or thresholds to distinguish wounds being inflamed from wounds being not inflamed. Similarly, the predetermined concentrations for the bacterial markers may be understood as limits or thresholds to distinguish wounds being colonized with metabolically active bacteria from wounds being not colonized with metabolically active bacteria.

In particular, the status of the wound is assessed as being inflamed if the one or more inflammatory markers are contained in the sample below the predetermined concentrations. If more than one inflammatory marker has been determined in the sample and only one of them falls below its predetermined concentration, this may already suffice to assess the wound as being inflamed.
On the other hand, the status of the wound may be assessed as being not inflamed if the one or more inflammatory markers are contained in the sample in or above the predetermined concentrations. For this assessment usually every determined inflammatory marker has to be contained in the sample in or above its predetermined concentration.
In particular, the predetermined concentration for a specific inflammatory marker may be chosen according to that (minimum) concentration which may be present in a wound being not inflamed. In the following, such predetermined concentrations for the inflammatory markers glucose and eotaxin-1 are exemplarily mentioned.

The predetermined concentration for glucose may be 1.35 to 4.05 mM, in particular 2.7 mM. The predetermined concentration for eotaxin-1 may be 110 to 330 pg/ml, in particular 220 pg/ml.

The status of the wound may then be assessed as being colonized with metabolically active bacteria if the one or more bacterial markers are contained in the sample above the predetermined concentrations. If more than one bacterial marker has been determined in the sample and only one or some of them exceed their predetermined concentration, this may already suffice to assess the wound as being colonized with metabolically active bacteria. On the other hand, the status of the wound may be assessed as being not colonized with metabolically active bacteria if the one or more bacterial markers are contained in the sample in or below the predetermined concentrations. For this assessment usually every determined bacterial marker has to be contained in the sample in or below its predetermined concentration.
The bacterial markers according to the present invention are usually completely absent in wounds being not colonized with metabolically active bacteria. Thus, reliable results regarding the bacterial colonization may be achieved if the predetermined concentration for one particular bacterial marker, some bacterial markers or preferably even all bacterial markers determined in the sample is simply set to zero. In this case, the sole presence or absence of the bacterial markers is decisive on whether the wound is assessed as being colonized with metabolically active bacteria or not. That is, the status of the wound may be assessed as being colonized with metabolically active bacteria if the one or more bacterial markers are present in the sample and, on the other hand, may be assessed as being not colonized with metabolically active bacteria if the one or more bacterial markers are absent in the sample.

The method of the present invention may also comprise additional steps. For example, the method may further comprise a step of determining a pH value of the sample. If the determined pH value is 7.1 or higher, in particular 7.1 to 8.2, this may indicate a poorly healing or even non-healing status of the wound such as typical for a chronic wound. The reason for the (slightly) alkaline pH value might be a bacterial colonization of the wound. Therefore, such an alkaline pH value may be an additional hint for a colonization of the wound with metabolically active bacteria. Consequently, if one or more bacterial markers are determined in the sample and the pH value of the sample is alkaline as mentioned before, the examined wound may be assessed as being colonized with metabolically active bacteria with a very high degree of certainty. In addition, the bacterial colonization may then be assessed as being very serious. In the end, the accuracy of the present method may be increased by the pH value determination. Nevertheless, the pH value determination is just an auxiliary measure as the pH of a wound is also influenced by factors other than bacterial colonization.

In a particularly preferred embodiment of the present invention the method further comprises a step of assessing if an anti-inflammatory treatment and/or an antibacterial treatment of the wound is indicated or not, based on the status of the wound. This may improve the healing process of the wound, which is also an aim underlying the present invention. Moreover, unnecessary or even detrimental treatments of the wound may be prevented by this further assessment.

For example, the anti-inflammatory treatment may be indicated for a wound being inflamed and not colonized with metabolically active bacteria. Such a wound status may occur in a patient suffering from an autoimmune disease (for instance rheumatoid arthritis). However, the anti-inflammatory treatment may be not indicated for a wound being inflamed and colonized with metabolically active bacteria (which is the typical example of an infected wound), since in this case the inflammation might be beneficial to fight the bacterial infection. In addition, the anti-inflammatory treatment may be obviously not indicated for a wound which has been assessed by the present diagnostic method as being not inflamed.

Advantageously, the antibacterial treatment may be indicated for a wound being inflamed and colonized with metabolically active bacteria. As mentioned above, a simultaneous application of an anti-inflammatory treatment in the case of such an infected wound may be detrimental for wound healing. On the other hand, the antibacterial treatment may be not indicated for a wound being not inflamed and colonized with metabolically active bacteria (that is a wound being not inflamed, but being colonized with metabolically active bacteria). In this case, the lack of an inflammation within the wound might indicate that the bacterial colonization is not harmful for the patient and does not impede the wound healing process. Therefore, no antibacterial treatment would then be required. Furthermore, the antibacterial treatment may be not indicated for a wound being not colonized with metabolically active bacteria.

Preferably, the anti-inflammatory treatment comprises the administration of a glucocorticoid and/or a non-steroidal anti-inflammatory drug (NSAID) to the patient. The antibacterial treatment preferably comprises the administration of an antibiotic and/or an antiseptic to the patient. The glucocorticoid, the NSAID and the antibiotic may be administered locally or systemically to the patient. The antiseptic (for example octenidine) is usually only administered locally to the patient. Glucocorticoids, NSAIDs, antibiotics and antiseptics suitable in connection with the present invention are known in the prior art.

In general, the concentrations of the markers may be determined by an enzymatic, immunological (in particular an ELISA or a lateral flow test), colorimetric, fluorimetric, radioactive and/or spectrophotometric analytical method, wherein the enzymatic and immunological analytical methods are particularly preferred. According to a particular advantageous embodiment of the present invention the concentrations of the markers are determined by a dipstick type test. Such a test is easy to handle, can be carried out quickly and may even be performed by the patient alone. The dipstick type test may also comprise a hand-held electronic unit into which the dipstick (test strip) is inserted for evaluation. For example, the used technology may be similar to that one of hand-held blood glucose measurement systems.

The invention also relates to a device for assessing a status of a wound of a patient, comprising
i. means for determining a concentration of one or more inflammatory markers selected from glucose and eotaxin-1 in a sample from the wound, wherein the sample comprises or consists of wound fluid and the one or more inflammatory markers may indicate an inflammation within the wound,
ii. means for determining a concentration of one or more bacterial markers in the sample, wherein the one or more bacterial markers may indicate a colonization of the wound with metabolically active bacteria,
iii. means for indicating the concentrations of the one or more inflammatory markers and the one or more bacterial markers.

The device may also indicate or assess the status of the wound based on the determined concentrations of the one or more inflammatory markers and the one or more bacterial markers. In doing so, the status of the wound is preferably indicated or assessed by the device as being inflamed or not inflamed and as being colonized with metabolically active bacteria or not colonized with metabolically active bacteria.

As an example, the device may comprise a dipstick (test strip) and, preferably, in addition a hand-held electronic measurement unit for the dipstick. In other words, the device may be constituted by a dipstick (test strip) and preferably a hand-held electronic measurement unit for the dipstick, wherein the dipstick itself or possibly in combination with the hand-held electronic measurement unit comprises the above mentioned three means for determining and indicating the concentrations of the markers.
The device may be also provided in the form of a kit. For example, the kit may comprise one or more of said dipsticks and one or more syringes or pipettes for collecting a sample of wound fluid.

Suitable means for determining and indicating the concentrations of the markers are known to the person skilled in the art. Preferably, said means are components of an enzymatic and/or immunological analytical assay system (for instance enzymes, antibodies and/or dyes from an ELISA or a lateral flow test adapted to detect the inflammatory and bacterial markers), which may be located on the dipstick mentioned before. Suitable detection technology for the present invention can also be found in GB 2 323 166 A. This British patent application discloses a diagnostic sheet for mapping the condition of a wound, which is selectively reactive over at least part of its area with one or more analytes present in the wound fluid. The sheet may be placed directly onto the wound for mapping or may have a further wound contacting absorbent layer. The sheet typically contains an immuno binding reagent for the analyte of interest in the wound fluid, e.g. a monoclonal antibody, or may contain a colorimetric, chemiluminescent or fluorogenic substrate. The sheet may comprise gelatin, cellulose, starch, gums or polypropylene etc.

The device may be adapted to perform a method according to any one of the previously mentioned embodiments. Therefore, the features of the previously mentioned embodiments of the method according to the present invention also apply for the device according to the present invention.

In connection with the device, a wound dressing is also claimed. The wound dressing comprises the aforementioned device. Such a wound dressing may protect the wound, improve wound healing and at the same time indicate the status of the wound. The diagnostic method of the present invention may be performed with such a wound dressing directly on the body of the patient, which may be very effective, simple and timesaving. Finally, a use of one or more inflammatory markers selected from glucose and eotaxin-1 in combination with one or more bacterial markers for assessing a status of a wound of a patient is claimed. The one or more inflammatory markers may indicate an inflammation within the wound. The one or more bacterial markers may indicate a colonization of the wound with metabolically active bacteria. The one or more inflammatory markers as well as the one or more bacterial markers are contained in a sample from the wound comprising or consisting of wound fluid.

The use may occur by performing the method according to any one of the previously mentioned embodiments. Therefore, the features of the previously mentioned embodiments of the method according to the present invention also apply for the use according to the present invention.

The following examples refer to preferred embodiments of the invention. They are meant to illustrate the present invention, but do not limit the scope of the present invention in any way.

### First example of use (test strip)

Home-care nurses face many times patients with pressure ulcers that might be only inflamed, and not needing extra care, or, worst, being infected and needing antibiotics or further hospital assistance care to avoid further complications. By evaluating the status of the wound with a diagnostic device according to a preferred embodiment of the present invention, the nurse will either take a sample of wound fluid with a syringe or disposable pipette and apply small quantities of the wound fluid to a test strip. In the case of wounds with enough exudate, the nurse can also apply the wound fluid to the test strip by contacting the test strip with the wound surface for a sufficient time. The test strip is adapted to generate a color pattern indicating a certain concentration level of at least one inflammatory marker and the presence or absence of at least one bacterial marker in the wound fluid. For example, the test strip may be reactive to glucose and/or eotaxin-1 as inflammatory marker(s) and ammonia and/or alkaline phosphatase as bacterial marker(s). Based on the concentration level and number of inflammatory and/or bacterial markers present in the wound fluid, the nurse will be able to quickly make an appropriate treatment decision, avoiding for example the use of antibiotics when it is not needed.

### Second example of use (laboratory test)

A health care professional in a hospital (inpatient or outpatient department) needs to decide whether a wound with clinical signs of infection requires antibiotic treatment or not. A swab is taken and send to the central laboratory. There the swab is rehydrated with an appropriate solution and tested for the amount of inflammatory mediators (glucose and/or eotaxin-1) and bacterial metabolites (for example acetoin, acetone, ammonia and/or nitrite). Different to current practice of culturing bacteria from swabs the presence of bacterial metabolites indicates bacterial metabolism and is independent of the cultivation time of the bacteria (allowing a quick test result) and bacterial viability on the swab, which might decay during transport to the laboratory.

### Third example (detection of alkaline phosphatase)

A chromogenic substrate (e.g. 4-nitrophenylphosphate-2CHA) with a phosphorylated end is immobilized on the surface of the test strip. By applying the wound fluid (either by the direct absorption of the fluid on the wound or by applying the wound fluid extracted from the wound) to the labeled area of the test strip, the bacterial alkaline phosphatase enzyme (when present in the wound fluid) will cleave the phosphate end of the substrate and release a chromogenic product which leads to the formation of a color change visible with the naked eye.

### Fourth example (detection of coagulases)

Latex particles coated with fibrinogen are immobilized to the test strip to detect clumping factor of *Staphylococcus aureus.* By wetting the test strip with wound fluid, a rapid agglutination occurs and can be observed with the naked eye.

### Fifth example (detection of coagulases)

After soaking the test strip with wound fluid, the nurse in charge adds a drop of a reagent containing latex particles coated with fibrinogen to the test strip labeled area and a rapid agglutination occurs through the interaction of fibrinogen and clumping factor. This agglutination is visible with the naked eye.

### Sixth example (detection of nitrate reductase)

The nitrate reduction test determines the production of the enzyme nitrate reductase, which results in the reduction of nitrate (NO₃⁻) to nitrite (NO₂⁻). Nitrate is immobilized to the test strip on a specific area labeled for the detection of nitrate reductase. After soaking or adding the wound fluid to the test strip, the nurse in charge adds a drop of a reagent containing sulfanilic acid and alfa-naphthylamine to produce prontosil, a red precipitate, which formation is visible with the naked eye.

## Claims

1. Method for assessing a status of a wound of a patient, comprising the steps of
i. determining a concentration of one or more inflammatory markers selected from glucose and eotaxin-1 in a sample from the wound, wherein the sample comprises or consists of wound fluid and the one or more inflammatory markers may indicate an inflammation within the wound,
ii. determining a concentration of one or more bacterial markers in the sample, wherein the one or more bacterial markers may indicate a colonization of the wound with metabolically active bacteria,
iii. assessing the status of the wound based on the concentrations of the one or more inflammatory markers and the one or more bacterial markers, wherein the status of the wound is assessed as being inflamed or not inflamed and as being colonized with metabolically active bacteria or not colonized with metabolically active bacteria.

2. Method of claim 1, wherein the one or more bacterial markers are selected from a coagulase, an enzyme, a metabolite, a siderophore, a signaling molecule and a virulence factor.

3. Method of claim 2, wherein the coagulase is staphylocoagulase, von Willebrand factor binding protein, clumping factor A or clumping factor B.

4. Method of claim 2, wherein the enzyme is alkaline phosphatase or nitrate reductase.

5. Method of claim 2, wherein the metabolite is acetoin, acetone, ammonia, nitrate or nitrite.

6. Method of claim 2, wherein the siderophore is acinetobactin, aerobactin, baumannoferrin, enterobactin, fimsbactin, staphyloferrin A, staphyloferrin B, pyochelin, pyoverdine, salmochelin or yersiniabactin.

7. Method of claim 2, wherein the signaling molecule is a quorum sensing molecule, in particular indole or autoinducer-2.

8. Method of claim 2, wherein the virulence factor is a quorum sensing regulated molecule, in particular alkaline protease, alpha-toxin, γ-hemolysin, LasA protease, LasB elastase, LecA lectin, LukDE, LukGH (LukAB), panton-valentine-leukocidin (PVL), a phenol soluble modulin (PSM), pyocyanin or a rhamnolipid.

9. Method of any one of the preceding claims, wherein the status of the wound is assessed by comparing the determined concentrations of the one or more inflammatory markers and the one or more bacterial markers with predetermined concentrations for the one or more inflammatory markers and the one or more bacterial markers, wherein
- the status of the wound may in particular be assessed as being inflamed if the one or more inflammatory markers are contained in the sample below predetermined concentrations, and/or
- the status of the wound may in particular be assessed as being not inflamed if the one or more inflammatory markers are contained in the sample in or above predetermined concentrations, and/or
- the status of the wound may in particular be assessed as being colonized with metabolically active bacteria if the one or more bacterial markers are contained in the sample above predetermined concentrations, and/or
- the status of the wound may in particular be assessed as being not colonized with metabolically active bacteria if the one or more bacterial markers are contained in the sample in or below predetermined concentrations.

10. Method of any one of the preceding claims, wherein the method further comprises a step of determining a pH value of the sample, wherein the pH value may indicate a poorly healing or non-healing wound.

11. Method of any one of the preceding claims, wherein the method further comprises a step of assessing if an anti-inflammatory treatment and/or an antibacterial treatment of the wound is indicated or not based on the status of the wound.

12. Method of any one of the preceding claims, wherein the concentrations of the markers are determined by an enzymatic, immunological, colorimetric, fluorimetric, radioactive and/or spectrophotometric analytical method.

13. Method of any one of the preceding claims, wherein the concentrations of the markers are determined by a dipstick type test.

14. Device for assessing a status of a wound of a patient, comprising
i. means for determining a concentration of one or more inflammatory markers selected from glucose and eotaxin-1 in a sample from the wound, wherein the sample comprises or consists of wound fluid and the one or more inflammatory markers may indicate an inflammation within the wound,
ii. means for determining a concentration of one or more bacterial markers in the sample, wherein the one or more bacterial markers may indicate a colonization of the wound with metabolically active bacteria,
iii. means for indicating the concentrations of the one or more inflammatory markers and the one or more bacterial markers,
wherein the device may in particular be adapted to perform a method of any one of the claims 1 to 13.

15. Wound dressing comprising a device of claim 14.

16. Use of one or more inflammatory markers selected from glucose and eotaxin-1 in combination with one or more bacterial markers for assessing a status of a wound of a patient, wherein the one or more inflammatory markers may indicate an inflammation within the wound and wherein the one or more bacterial markers may indicate a colonization of the wound with metabolically active bacteria, wherein the use may in particular occur by performing the method of any one of the claims 1 to 13.
